# FASCICULE DE BREVET EUROPEEN

(11) **EP 2 105 092 B1**
(45) Date de publication et mention de la délivrance du brevet: **14.08.2013**
(21) Numéro de dépôt: 09156381.7
(22) Date de dépôt: 27.03.2009
(51) Int. Cl.: A61B 5/18

(54) **Procédé et système de limitation d'usage d'un véhicule en fonction de l'état de vigilance d'un utilisateur.**
Verfahren und Gerät um die Nutzung eines Fahrzeuges zu limitieren abhängig von der Aufmerksamkeit vom Benutzer
Method and system for limiting the use of a vehicle as a function of the awareness state of the user

(30) Priorité: 28.03.2008 FR 0801721
(43) Date de publication de la demande: 30.09.2009
(73) Titulaire: Valeo Systèmes Thermiques, 78321 Le Mesnil Saint Denis Cedex (FR)
(72) Inventeur: Giraud, Frédéric, 78610 Le Perray en Yvelines (FR); Martinell, Amanda, 60128 Plailly (FR); Gehin, Frédéric, 94100 Saint Maur de Fosses (FR)
(74) Mandataire: Léveillé, Christophe

(56) Documents cités:
- EP-A- 0 260 931
- FR-A- 2 639 533
- GB-A- 2 420 631
- US-A- 3 665 447
- US-A- 3 879 705

## Description

La présente invention concerne un procédé et un système de limitation d'usage d'un véhicule en fonction de l'état de vigilance d'un utilisateur dudit véhicule.

L'invention trouve une application particulièrement avantageuse dans le domaine de la sécurité routière.

Dans plus en plus de pays, de nombreux constructeurs automobiles s'interrogent aujourd'hui sur l'opportunité d'équiper leurs véhicules, que ce soit en série, en option ou en après-vente, de détecteurs de substances pouvant affecter la vigilance des utilisateurs, comme l'alcool, les drogues ou les médicaments.

Ils sont cependant confrontés à un certain nombre de problèmes.

Il est difficile en effet de détecter ces trois familles de produits avec un seul et même système qui soit en plus simple d'utilisation.

Les éthylotests et autres systèmes destinés à détecter les conducteurs sous l'emprise de l'alcool ou de stupéfiants sont délicats, ont une durée de vie limitée et nécessitent un entretien conséquent.

D'autre part, les systèmes de contrôle d'alcoolémie, tels que les éthylotests, mesurent le taux d'alcool, mais pas l'état réel d'intoxication, lequel dépend de la physiologie de chacun. De plus, le taux d'alcool fourni par les éthylotests est le taux de l'alcool contenu dans l'air expiré par la personne et non celui de l'alcool réellement contenu dans son sang. Il en résulte des possibilités d'erreur lorsque la personne contrôlée présente un taux d'alcool beaucoup plus élevé dans l'air qu'elle expire que dans son sang. C'est le cas par exemple lorsqu'une personne a bu un sirop contre la toux ou ingéré un bonbon alcoolisé, ou encore lorsque le véhicule a été nettoyé avec un solvant.

Ces systèmes sont également très pénalisants car ils interdisent le démarrage du moteur alors que seule la conduite en état d'ivresse est interdite. Or, il est des situations où une personne, même dans un état alcoolique, ait besoin de démarrer le moteur du véhicule, ne serait-ce que pour mettre le chauffage ou la climatisation en marche.

Enfin, les systèmes actuellement proposés sont facilement falsifiables. S'agissant en général d'éthylotests qui, comme on vient de le voir, analysent le taux d'alcool dans l'air expiré, la personne dont l'alcoolémie est contrôlée peut très bien être le conducteur mais aussi un passager se substituant au conducteur le temps du contrôle.

FR 2 639 533 décrit un procédé et un système selon les preambles des revendications 1 et 14.

Aussi, un but de l'invention est de proposer un procédé de limitation d'usage d'un véhicule en fonction de l'état de vigilance d'un utilisateur dudit véhicule, qui permettrait d'apporter une solution aux différentes difficultés qui viennent d'être mentionnées à propos des systèmes connus de l'état de la technique, et, en premier lieu, l'indépendance du procédé en regard de l'origine d'un éventuel défaut de vigilance constaté, à savoir l'alcool, les drogues ou certains médicaments.

Ce but est atteint, conformément à l'invention, du fait que ledit procédé comprend les étapes consistant à :
- soumettre ledit utilisateur à au moins un test perceptif,
- comparer le résultat dudit test perceptif à une échelle de niveaux de risque relativement à la conduite du véhicule et en déduire le niveau de risque présenté par l'utilisateur,
- informer l'utilisateur du niveau de risque qu'il présente.

Ainsi, on comprend que, s'appuyant sur un test faisant appel aux facultés réelles de perception de l'utilisateur, un tel procédé puisse être efficace en toutes circonstances pour détecter une baisse de vigilance de ce même utilisateur au moment où il s'apprête à conduire le véhicule, et ceci quelle que soit la cause de la baisse de vigilance constatée : alcool, drogues ou médicaments. En ce sens, on remarquera que l'invention s'étend avantageusement à la baisse de vigilance due à l'état de fatigue du conducteur.

En plus de son caractère universel, le procédé selon l'invention est de mise en oeuvre très simple, ergonomique, sans entretien ni limitation de durée, contrairement aux éthylotests et autres détecteurs de drogues par exemple.

D'autre part, le résultat du test perceptif est indépendant de la physiologie des individus sur lesquels il est effectué, c'est-à-dire de leur façon de réagir face à l'absorption de produits susceptibles d'affecter leur vigilance, ainsi que de la fiabilité des mesures du taux d'alcool réellement contenu dans le sang.

Selon un premier mode de réalisation, ledit test perceptif est un test de perception sensorielle.

Selon un second mode de réalisation, ledit test perceptif est un test de temps de réaction.

En complément du caractère purement informatif à destination de l'utilisateur sur son niveau de risque et d'éventuelles recommandations de limitation d'usage qui l'accompagnent, l'invention prévoit que ledit procédé comprend en outre une étape consistant à associer à au moins un niveau de risque au moins une action technique de limitation d'usage du véhicule. Cette disposition a bien entendu un but plus coercitif, visant à empêcher par des moyens techniques l'utilisation du véhicule lorsque le niveau de risque atteint par la personne soumise au test perceptif l'exige pour des raisons de sécurité.

Dans ce cas, l'utilisateur peut être également informé de l'action technique de limitation associée au niveau de risque qu'il présente.

Selon un mode de réalisation, ladite action technique concerne l'immobilisation du véhicule.

En particulier, l'action technique concernant l'immobilisation du véhicule est le blocage de la direction ou le calage du moteur si une vitesse est enclenchée.

Conformément à l'invention, ladite étape de soumettre ledit utilisateur à au moins un test perceptif est réalisée à la suite d'une étape préalable consistant à effectuer par l'utilisateur une demande de démarrage du moteur du véhicule et à empêcher le démarrage dudit moteur.

Afin de remédier à l'inconvénient de l'art antérieur d'empêcher tout démarrage du moteur si le test d'alcoolémie par exemple se révèle positif, l'invention propose que le démarrage du moteur du véhicule est disponible à la suite dudit test perceptif, quel que soit le niveau de risque présenté par l'utilisateur.

Dans ce contexte, l'invention prévoit que, pour un niveau de risque dit « sans risque », le démarrage du moteur du véhicule est effectué automatiquement à la suite dudit test perceptif, alors que, pour au moins un niveau de risque dit « à risque », le démarrage du moteur du véhicule est effectué à la suite dudit test perceptif sur une nouvelle action volontaire de l'utilisateur.

Avantageusement, ladite action volontaire consiste à effectuer à nouveau une demande de démarrage du moteur du véhicule.

Afin d'éviter les possibilités de falsification mentionnées plus haut, le procédé conforme à l'invention comprend en outre un test de vérification de l'identité entre le conducteur du véhicule et l'utilisateur soumis audit test perceptif.

Selon l'invention, ledit test de vérification concerne, séparément ou en combinaison, l'occupation du siège conducteur, l'ouverture de la portière conducteur, le verrouillage de la ceinture de sécurité conducteur, la présence du conducteur.

De préférence, ledit test de vérification est effectué automatiquement après ledit test perceptif, le véhicule étant à l'arrêt.

En cas de test de vérification négatif, le moteur du véhicule est arrêté et/ou le véhicule est immobilisé.

Selon une caractéristique avantageuse, le procédé selon l'invention comprend une étape initiale consistant à fournir un code spécifique pour l'exécution obligatoire du test perceptif.

On obtient ainsi que, même s'il n'est pas obligatoire, le procédé de limitation d'usage conforme à l'invention puisse être néanmoins mis en oeuvre à volonté grâce à la saisie dudit code spécifique. De cette manière, des parents peuvent prêter leur véhicule en toute confiance à de jeunes adultes, sachant que ces derniers ne pourront le conduire que si leur état de vigilance s'avère satisfaisant à la suite du test perceptif qu'ils auront obligatoirement subi.

L'invention concerne également un système de limitation d'usage d'un véhicule en fonction de l'état de vigilance d'un utilisateur dudit véhicule, selon la revendication 14.

Selon l'invention, ladite unité de gestion est également apte à associer à au moins un niveau de risque au moins une action technique de limitation d'usage du véhicule.

Le système selon l'invention comprend en outre un module de commande d'au moins un organe du véhicule apte à exécuter ladite action technique associée au niveau de risque présenté par l'utilisateur.

Enfin, avantageusement, le système selon l'invention comprend une interface de paramétrage apte à recevoir un code spécifique pour l'exécution obligatoire du test perceptif, ceci pour les raisons qui ont été exposées plus haut.

La description qui va suivre en regard des dessins annexés, donnés à titre d'exemples non limitatifs, fera bien comprendre en quoi consiste l'invention et comment elle peut être réalisée.
La figure 1 est un schéma d'un système de limitation d'usage d'un véhicule, conforme à l'invention.
La figure 2 représente un exemple d'échelle de niveaux de risque utilisée par l'invention.
La figure 3 est un organigramme illustrant le déroulement d'un procédé de limitation d'usage d'un véhicule, conforme à l'invention.

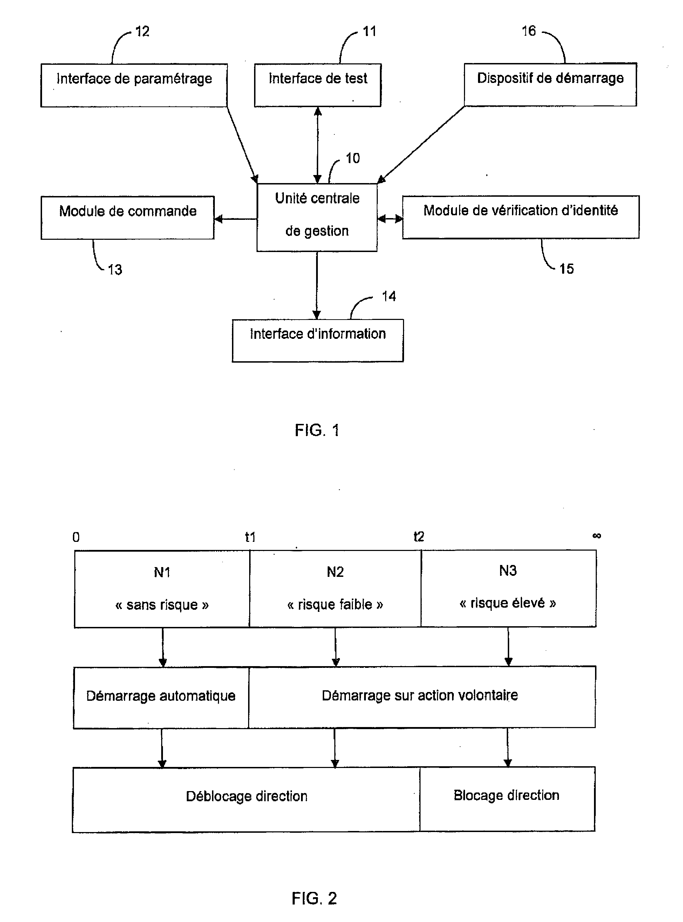

Sur la figure 1 est représenté un système de limitation d'usage d'un véhicule en fonction de l'état de vigilance d'un utilisateur dudit véhicule.

Ce système comprend une interface 11 de test apte à soumettre un utilisateur du véhicule assis sur le siège conducteur à au moins un test perceptif destiné à estimer l'état de vigilance de cet utilisateur et à en déduire le niveau de risque qu'il présente relativement à la conduite d'un véhicule.

Le test perceptif est effectué dès qu'une unité centrale 10 de gestion reçoit du dispositif 16 de démarrage du moteur du véhicule un signal de demande de démarrage provenant de l'utilisateur.

En pratique, après avoir mis le contact du véhicule, l'utilisateur commande le démarrage du moteur de manière usuelle en tournant la clé de contact. Dans le cadre de l'invention, cette demande de démarrage est enregistrée par l'unité centrale 10 de gestion, mais cette dernière ne permet pas au dispositif 16 de procéder au démarrage immédiat du moteur, afin d'effectuer au préalable le ou les tests perceptifs prévus. L'invention s'étend bien entendu au cas où la clé de contact est remplacée par un dispositif dit « mains libres » constitué par un transpondeur porté par l'utilisateur et un bouton de démarrage situé par exemple sur la planche de bord du véhicule.

Les composants électroniques de l'unité centrale 16 de gestion sont de préférence intégrés dans un calculateur déjà existant, notamment un calculateur lié au démarrage, comme le calculateur du système ESCL de blocage de la direction (« Electrical Steering Column Lock » en anglais), le calculateur du système « mains libres », ou encore le calculateur du boîtier de démarrage.

L'interface 11 de test peut comporter un écran ou un ensemble de pictogrammes indiquant à l'utilisateur la séquence des instructions de test qu'il devra exécuter.

Concrètement, le test perceptif doit satisfaire de préférence à un certain nombre de critères parmi les critères suivants :
- Il doit être aléatoire et prendre à chaque fois une forme différente.
- Il peut être envisagé une graduation de la difficulté dans le test, celui-ci commençant par une première étape qui, si elle est validée, démontre au pire un défaut de vigilance à la limite de l'acceptable, suivie d'une deuxième étape, plus sévère, qui, si elle est validée, démontre un défaut de vigilance faible.
- Il doit prendre un temps très court, pas plus de 10 s par exemple, qui est le temps maximum accordé à une fonction préchauffage de moteur diesel dans les pires conditions.
- Il doit être susceptible d'une quantification sur une échelle afin de pouvoir définir des niveaux de risque en fonction de l'état de vigilance estimé à partir du test.
- Il doit être capable de discriminer les défauts de vigilance de manière absolue et universelle par rapport à la diversité des personnes, ou s'adapter à l'utilisateur, lequel est alors reconnu via un identifiant ou par une caméra intérieure au véhicule. C'est pourquoi les tests de nature cognitive ou de perception sont particulièrement intéressants.
- Il doit être ergonomiquement très proche d'une situation d'accident de manière à ce que le résultat du test soit convaincant et persuasif pour l'utilisateur (temps de réaction au freinage par exemple).

Selon un premier mode de réalisation, ledit test perceptif est un test de perception sensorielle. On peut citer par exemple un test de perception simultanée de deux pictogrammes placés à gauche et à droite de l'utilisateur. Ce test est bien adapté au défaut de vigilance due à l'alcoolémie, l'alcool réduisant le champ visuel des individus.

Selon un second mode de réalisation, ledit test perceptif est un test de temps de réaction.

Dans cette catégorie de tests, on peut mentionner les exemples suivants :
- Taper une suite de chiffres sur le clavier du téléphone du véhicule en moins de x secondes.
- Suivre une cible sur l'écran multi-fonctions du véhicule pendant x secondes en manoeuvrant le volant.
- Appuyer sur le frein du véhicule dés l'apparition d'un signe quelconque sur l'écran ou le combiné d'instruments. Le signe apparaît une ou plusieurs fois (deux fois par exemple : 1 première fois pour prévenir du début du test et une deuxième fois pour faire effectivement le test) à des intervalles irréguliers, et une moyenne des temps de réaction est calculée.
- Enfoncer en un temps donné une série de boutons colorés dans le même ordre qu'un ordre aléatoire dans lequel ils ont été allumés alternativement.

Les résultats du test sont fournis par l'interface 11 à l'unité centrale 10 de gestion qui en effectue l'analyse en les comparant par exemple à une échelle de niveaux de risque relativement à la conduite du véhicule afin d'en déduire le niveau de risque présenté par la personne testée.

La figure 2 donne un exemple d'une telle échelle de niveaux de risque.

Dans cet exemple, l'échelle comporte trois niveaux de risque définis par des valeurs t1 et t2 du temps de réaction à un test donné :
- pour des valeurs de temps de réaction comprises entre 0 et t1, le niveau N1 est dit « sans risque », l'état de vigilance de l'utilisateur étant considéré comme compatible avec la conduite d'un véhicule,
- pour des valeurs de temps de réaction supérieures à t1, les niveaux N2 et N3 sont dits « à risque ».

* Le niveau N2 défini pour des temps de réaction compris entre t1 et t2 est « à risque faible ». L'utilisateur peut démarrer s'il le désire, mais il lui est recommandé d'attendre quelques minutes ou quelques heures et de repasser le test.
* Le niveau N3 défini pour des temps de réaction supérieurs à t3 est « à risque élevé », l'utilisateur n'étant pas considéré en état de conduire le véhicule.

Dans un cadre purement informatif de l'invention, seul le niveau de risque, N1, N2 ou N3, qu'il présente est communiqué à l'utilisateur par une interface 14 d'information sur un écran, un ensemble de pictogrammes, par synthèse vocale ou par un message sonore.

Eventuellement, de manière plus coercitive, l'unité centrale 10 de gestion peut associer à chaque niveau de risque une action technique de limitation d'usage du véhicule et transmettre à un module 13 de commande un ordre d'exécution de l'action de limitation décidée par l'unité centrale 10. L'utilisateur est par ailleurs informé par l'interface 14 de cette action de limitation.

D'une façon générale, ladite action technique de limitation d'usage est une action concernant l'immobilisation du véhicule. On préférera utiliser le blocage de la direction ESCL, car il présente l'avantage de permettre le démarrage tout en interdisant au véhicule de rouler ce qui permet de maintenir des conditions de confort acceptables dans le véhicule. On peut également caler le moteur si une vitesse est enclenchée. Au pire, le système anti-démarrage supprimera la possibilité de lancer le moteur.

Toutes ces dispositions techniques complémentaires sont résumées sur le diagramme de la figure 2.

On peut voir sur cette figure que, s'agissant du niveau N1 « sans risque », le démarrage du moteur est réalisé automatiquement en sortie du test, sans aucune action à effectuer de la part de l'utilisateur. La direction est également débloquée. Le conducteur peut normalement utiliser le véhicule.

Dans le cas des niveaux N2 et N3 « à risque », la possibilité de démarrer le moteur du véhicule est laissée à l'utilisateur à condition qu'il effectue une action volontaire, comme le fait de demander à nouveau le démarrage du moteur du véhicule au moyen de la clé de contact ou du bouton de démarrage du système « mains libres ».

On comprend que, quel que soit le niveau de risque détecté, le démarrage du moteur est toujours disponible, contrairement aux systèmes basés sur les éthylotests par exemple, ce qui permet de pouvoir accéder à certaines fonctions du véhicule comme le chauffage, même si par ailleurs l'utilisateur n'est pas en état de conduire.

Par contre, la figure 2 montre que lorsque le niveau de risque a atteint le niveau N3 « à risque élevé », le véhicule est immobilisé, la direction restant bloquée, alors que pour les niveaux N1 et N2 le véhicule peut être utilisé à la suite du déblocage de la direction à l'issue du test perceptif.

Comme le montre la figure 1, le système selon l'invention comprend en outre un module 15 destiné à vérifier l'identité entre la personne ayant subi le test et le conducteur du véhicule, ceci afin d'éviter toute substitution d'une personne ayant satisfait au test perceptif par une autre qui serait considérée « à risque ».

Le test de vérification d'identité effectué par le module 15 peut prendre des formes très diverses et concerner notamment :
- l'occupation du siège conducteur, au moyen d'une nappe biométrique de siège ou d'un simple capteur de pression installé dans le siège.
- l'ouverture de la portière conducteur, par un dispositif de gestion du mouvement des portières ou des serrures.
- le verrouillage de la ceinture de sécurité conducteur. Le test perceptif ne pourra être effectué que si la ceinture est bouclée et l'ensemble du processus se déroulera normalement que si la ceinture reste bouclée.
- la présence du conducteur, au moyen d'une caméra intérieure ou d'un capteur infrarouge dirigé vers le conducteur.

Comme on le verra en détail plus loin, ce test de vérification d'identité est effectué automatiquement préalablement ou en parallèle du test perceptif, le véhicule étant à l'arrêt. En cas de vérification négative, le moteur du véhicule est arrêté et/ou le véhicule est définitivement immobilisé, par exemple en bloquant la direction. Le test de vérification débute dès le déverrouillage du véhicule et se poursuit en parallèle du test de perception, cela permet notamment d'interrompre la séquence si un doute sur l'identité du "testé" survient (ceinture libérée, changement d'état du capteur de nappe siège, ouverture porte, ...).

Enfin, on peut noter sur la figure 1 la présence d'une interface 12 de paramétrage de l'ensemble du système. Cette interface 12 est notamment apte à recevoir un code spécifique destiné à rendre obligatoire l'exécution du test perceptif. Ainsi, le propriétaire du véhicule peut imposer à toute personne voulant utiliser le véhicule qu'elle soit soumise au test perceptif de vigilance avant de l'autoriser à prendre le volant.

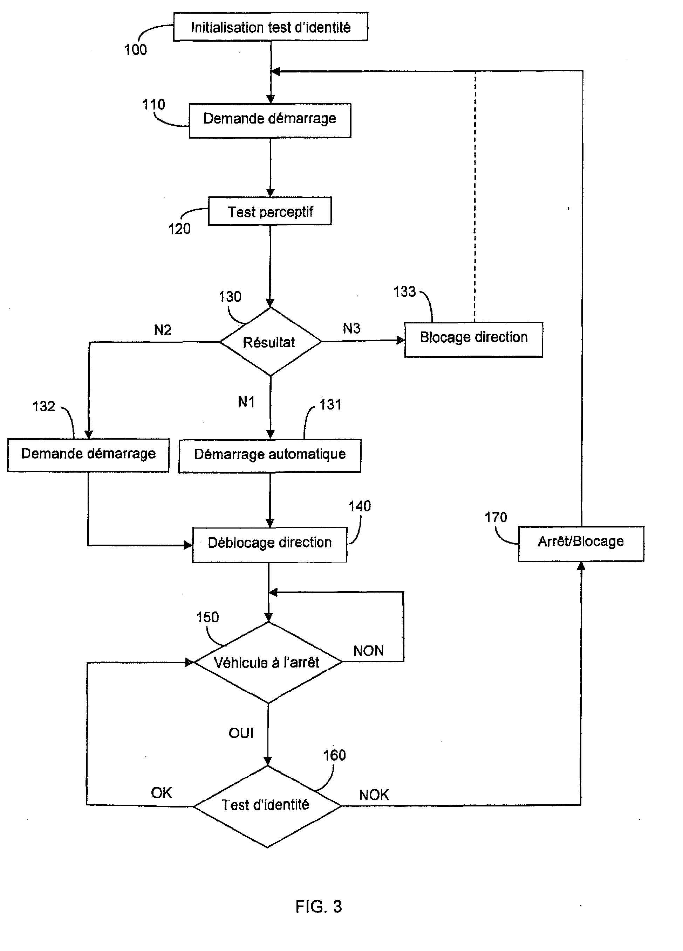

Sur la figure 3, ont été résumées les différentes étapes d'un procédé de limitation d'usage conforme à l'invention mettant en oeuvre l'échelle de risque à trois niveaux représentée sur la figure 2.

Comme le montre le mode de réalisation de la figure 3, le procédé débute par une phase 100 d'initialisation du module 15 de vérification d'identité consistant à enregistrer par exemple les données fournies par une nappe biométrique placée sur le siège conducteur.

Ensuite, l'utilisateur demande en 110 le démarrage du moteur à l'aide de la clé de contact ou du bouton de démarrage « mains libres ».

Le dispositif 16 de démarrage du véhicule transmet cette information à l'unité centrale 10 de gestion qui déclenche aussitôt en 120 le processus de test perceptif de vigilance à exécuter par l'interface 11 de test, et ceci sans que le démarrage du moteur, pourtant demandé en 110, ne soit effectué par le boîtier de démarrage du véhicule.

Les résultats du test établis par l'interface 11 sont analysés en 130 par l'unité centrale 10. En application de l'échelle de niveaux de risque de la figure 2, trois sorties sont possibles.

Si le niveau de risque révélé par le test est le niveau N3 « à risque élevé », l'utilisateur n'est pas autorisé à conduire le véhicule, la direction étant maintenue bloquée (133) par le module 13 de commande sur ordre de l'unité centrale 10 de gestion. Cependant, comme cela a déjà été précisé plus haut, l'utilisateur a la possibilité de démarrer le moteur sur une nouvelle demande de démarrage s'il veut accéder à certaines fonctions du véhicule comme le chauffage par exemple.

Si le niveau de risque est le niveau N1 « sans risque », le moteur démarre automatiquement (131) et la direction est débloquée (140).

Si le niveau de risque est le niveau N3 « à risque faible », le moteur ne peut être démarré en 132 que sur une action volontaire de l'utilisateur, en général une nouvelle demande de démarrage au moyen de la clé de contact ou du bouton de démarrage « mains libres ». La direction est débloquée (140) et le véhicule peut être utilisé.

Un test 150 est effectué en permanence de manière à savoir si le véhicule est à l'arrêt. Si ce test est positif, le module 15 vérifie en 160 que le conducteur est bien la même personne que celle qui a subi le test perceptif en comparant par exemple les données fournies par la nappe biométrique à celles qui ont été préalablement enregistrées lors de la phase 100 d'initialisation. Si cette vérification s'avère négative, le moteur est automatiquement arrêté et/ou la direction bloquée, le véhicule ne pouvant être utilisé à nouveau que si un nouveau test perceptif est effectué. Si la vérification est positive, le test 150 de détection de l'arrêt du véhicule est repris.

## Revendications

1. Procédé de limitation d'usage d'un véhicule en fonction de l'état de vigilance d'un utilisateur dudit véhicule, ledit procédé comprend les étapes consistant à :
- soumettre ledit utilisateur à au moins un test perceptif (120),
- comparer (130) le résultat dudit test perceptif à une échelle de niveaux (N1, N2, N3) de risque relativement à la conduite du véhicule et en déduire le niveau de risque présenté par l'utilisateur,
- informer l'utilisateur du niveau de risque qu'il présente, **caractérisé en ce que** ladite étape de soumettre ledit utilisateur à au moins un test perceptif (120) est réalisée à la suite d'un signal de demande de démarrage du moteur du véhicule et d'empêcher le démarrage dudit moteur, et dans lequel le démarrage du moteur du véhicule est disponible à la suite dudit test perceptif, quel que soit le niveau (N1, N2, N3) de risque présenté par l'utilisateur.
- et **en ce que** le procédé comprend pour au moins un niveau (N2, N3) de risque dit « à risque » une étape de démarrage du moteur du véhicule effectuée à la suite dudit test perceptif (120) sur une action volontaire de l'utilisateur.

2. Procédé selon la revendication 1, dans lequel ledit test perceptif est un test de perception sensorielle.

3. Procédé selon la revendication 1, dans lequel ledit test perceptif est un test de temps de réaction.

4. Procédé selon l'une quelconque des revendications 1 à 3, comprenant en outre une étape consistant à associer à au moins un niveau (N1, N2, N3) de risque au moins une action technique de limitation d'usage du véhicule.

5. Procédé selon la revendication 4, dans lequel ladite action technique concerne l'immobilisation du véhicule.

6. Procédé selon la revendication 5, dans lequel l'action technique concernant l'immobilisation du véhicule est le blocage de la direction ou le calage du moteur.

7. Procédé selon l'une quelconque des revendications précédentes, dans lequel pour un niveau (N1) de risque dit « sans risque » le démarrage du moteur du véhicule est effectué automatiquement (131) à la suite dudit test perceptif (120).

8. Procédé l'une quelconque des revendications 1 à 7, dans lequel ladite action volontaire consiste à effectuer (132) à nouveau une demande de démarrage du moteur du véhicule.

9. Procédé selon l'une quelconque des revendications 1 à 8, comprenant en outre un test (160) de vérification d'identité entre le conducteur du véhicule et l'utilisateur soumis audit test perceptif (120).

10. Procédé selon la revendication 9, dans lequel ledit test (160) de vérification concerne, séparément ou en combinaison, l'occupation du siège conducteur, l'ouverture de la portière conducteur, le verrouillage de la ceinture de sécurité conducteur, la présence du conducteur.

11. Procédé selon l'une des revendications 9 ou 10, dans lequel ledit test (160) de vérification est effectué automatiquement préalablement ou en parallèle dudit test perceptif (120), le véhicule étant à l'arrêt.

12. Procédé selon la revendication 11, dans lequel en cas de test (160) de vérification négatif, le moteur du véhicule est arrêté et/ou le véhicule est immobilisé (170).

13. Procédé selon l'une quelconque des revendications 1 à 12, comprenant une étape initiale consistant à fournir un code spécifique pour l'exécution obligatoire du test perceptif (120).

14. Système de limitation d'usage d'un véhicule en fonction de l'état de vigilance d'un utilisateur dudit véhicule, comprennant :
- une interface (11) de test, apte à soumettre ledit utilisateur à au moins un test perceptif,
- une interface (14) d'information apte à informer l'utilisateur du niveau de risque qu'il présente.
- une unité (10) centrale de gestion, apte à comparer le résultat dudit test perceptif à une échelle de niveaux (N1, N2, N3) de risque relativement à la conduite du véhicule et en déduire le niveau de risque présenté par l'utilisateur, ledit système **caractérisé en ce que** ladite unité centrale (10) de gestion est apte à commander l'interface (11) de test sur réception d'un signal de demande de démarrage du moteur du véhicule, provenant d'un dispositif (16) de démarrage du véhicule, et **en ce qu'**il comprend:
- un module (15) de vérification d'identité entre le conducteur du véhicule et l'utilisateur soumis audit test perceptif.

15. Système selon la revendication 14, dans lequel ladite unité centrale (10) de gestion est également apte à associer à au moins un niveau de risque au moins une action technique de limitation d'usage du véhicule.

16. Système selon la revendication 15, dans lequel ladite interface (14) d'information est également apte à informer l'utilisateur de ladite action technique associée au niveau de risque qu'il présente

17. Système selon l'une des revendications 15 ou 16, comprenant en outre un module (13) de commande d'au moins un organe du véhicule apte à exécuter ladite action technique associée au niveau de risque présenté par l'utilisateur.

18. Système selon l'une quelconque des revendications 14 à 17, comprenant une interface (12) de paramétrage apte à recevoir un code spécifique pour l'exécution obligatoire du test perceptif.

## Patentansprüche

1. Verfahren zur Einschränkung der Nutzung eines Fahrzeugs in Abhängigkeit von dem Aufmerksamkeitszustand eines Benutzers des Fahrzeugs, wobei das Verfahren die Schritte aufweist, die aus Folgendem bestehen:
- den Benutzer mindestens einem Wahrnehmungstest (120) unterziehen,
- das Ergebnis des Wahrnehmungstests mit einer Gefahrenniveauskala (N1, N2, N3) in Verbindung mit dem Fahren des Fahrzeugs vergleichen (130) und daraus das Gefahrenniveau ableiten, das der Benutzer darstellt,
- den Benutzer über das Gefahrenniveau, das er darstellt, informieren, **dadurch gekennzeichnet, dass** der Schritt, bei dem der Benutzer mindestens einem Wahrnehmungstest (120) unterzogen wird, im Anschluss an ein Anlassanfragesignal des Motors des Fahrzeugs ausgeführt wird, und das Anlassen des Fahrzeugs zu verhindern, und bei dem das Anlassen des Motors des Fahrzeugs im Anschluss an den Wahrnehmungstest ungeachtet des Gefahrenniveaus (N1, N2, N3), das der Benutzer darstellt, verfügbar ist,
- und dass das Verfahren für mindestens ein so genanntes Gefahrenniveau "mit Risiko" (N2, N3) einen Anlassschritt des Motors des Fahrzeugs aufweist, der im Anschluss an den Wahrnehmungstest (120) auf eine absichtliche Aktion des Benutzers hin ausgeführt wird.

2. Verfahren nach Anspruch 1, bei dem der Wahrnehmungstest ein sinnlicher Wahrnehmungstest ist.

3. Verfahren nach Anspruch 1, bei dem der Wahrnehmungstest ein Reaktionszeittest ist.

4. Verfahren nach einem der Ansprüche 1 bis 3, das ferner einen Schritt aufweist, der darin besteht, mit mindestens einem Gefahrenniveau (N1, N2, N3) mindestens eine technische Nutzungseinschränkungsaktion des Fahrzeugs zu verbinden.

5. Verfahren nach Anspruch 4, bei dem die technische Aktion das Stillstellen des Fahrzeugs betrifft.

6. Verfahren nach Anspruch 5, bei dem die technische Aktion, die das Stillstellen des Fahrzeugs betrifft, das Blockieren der Lenkung oder das Stehenbleiben des Motors ist.

7. Verfahren nach einem der vorhergehenden Ansprüche, bei dem für ein so genanntes Gefahrenniveau (N1) "ohne Risiko" das Anlassen des Motors des Fahrzeugs automatisch (131) im Anschluss an den Wahrnehmungstest (120) erfolgt.

8. Verfahren nach einem der Ansprüche 1 bis 7, bei dem die absichtliche Aktion darin besteht, erneut eine Anlassanfrage des Motors des Fahrzeugs auszuführen (132).

9. Verfahren nach einem der Ansprüche 1 bis 8, das ferner einen Identitätsprüfungstest (160) zwischen dem Fahrer des Fahrzeugs und dem Benutzer, der dem Wahrnehmungstest (120) unterzogen wird, aufweist.

10. Verfahren nach Anspruch 9, bei dem der Prüfungstest (160) getrennt oder kombiniert das Besetzen des Fahrersitzes, das Öffnen der Fahrertür, das Verriegeln des Fahrersicherheitsgurts, die Gegenwart des Fahrers betrifft.

11. Verfahren nach einem der Ansprüche 9 oder 10, bei dem der Prüfungstest (160) automatisch vor oder parallel zu dem Wahrnehmungstest (120) ausgeführt wird, wobei das Fahrzeug stillsteht.

12. Verfahren nach Anspruch 11, bei dem in dem Fall eines negativen Prüfungstests (160) der Motor des Fahrzeugs gestoppt und/oder das Fahrzeug stillgestellt (170) wird.

13. Verfahren nach einem der Ansprüche 1 bis 12, das einen anfänglichen Schritt aufweist, der darin besteht, einen spezifischen Code für die zwingende Ausführung des Wahrnehmungstests (120) zu liefern.

14. System zur Einschränkung der Nutzung eines Fahrzeugs in Abhängigkeit von dem Aufmerksamkeitszustand eines Benutzers des Fahrzeugs, das Folgendes aufweist:
- eine Testschnittstelle (11), die geeignet ist, um den Benutzer mindestens einem Wahrnehmungstest zu unterziehen,
- eine Informationsschnittstelle (14), die geeignet ist, um den Benutzer über das Gefahrenniveau, das er darstellt, zu informieren,
- eine zentrale Verwaltungseinheit (10), die geeignet ist, um das Ergebnis des Wahrnehmungstests mit einer Gefahrenniveauskala (N1, N2, N3) in Verbindung mit dem Fahren des Fahrzeugs zu vergleichen und daraus das Gefahrenniveau, das der Benutzer darstellt, abzuleiten, wobei das System **dadurch gekennzeichnet ist, dass** die zentrale Verwaltungseinheit (10) geeignet ist, um die Testschnittstelle (11) bei Empfang eines Anlassanfragesignals des Motors des Fahrzeugs, das von einer Anlassvorrichtung (16) des Fahrzeugs kommt, zu steuern, und dass es Folgendes aufweist:
- ein Identitätsprüfmodul (15) zwischen dem Fahrer des Fahrzeugs und dem Benutzer, der dem Wahrnehmungstest unterzogen wird.

15. System nach Anspruch 14, bei dem die zentrale Verwaltungseinheit (10) auch geeignet ist, um mit mindestens einem Gefahrenniveau mindestens eine technische Nutzungseinschränkungsaktion des Fahrzeugs zu verbinden.

16. System nach Anspruch 15, bei dem die Informationsschnittstelle (14) auch geeignet ist, um den Benutzer über die mit dem Gefahrenniveau, das er darstellt, verbundene technische Aktion zu informieren.

17. System nach einem der Ansprüche 15 oder 16, das ferner ein Steuermodul (13) mindestens eines Organs des Fahrzeugs aufweist, das geeignet ist, um die mit dem von dem Benutzer dargestellten Gefahrenniveau verbundene technische Aktion auszuführen.

18. System nach einem der Ansprüche 14 bis 17, das eine Parametrierungsschnittstelle (12) aufweist, die geeignet ist, um einen spezifischen Code für die zwingende Ausführung des Wahrnehmungstests zu empfangen.

## Claims

1. Method of limiting use of a vehicle as a function of the state of vigilance of a user of the said vehicle, the said method comprises the steps consisting in:
- subjecting the said user to at least one perceptive test (120),
- comparing (130) the result of the said perceptive test with a scale of levels (N1, N2, N3) of risk in relation to the driving of the vehicle and deducing therefrom the level of risk presented by the user,
- informing the user of the level of risk that he presents,
**characterized in that** the said step of subjecting the said user to at least one perceptive test (120) is carried out subsequent to a signal requesting starting of the engine of the vehicle and to prevent the starting of the said engine, and in which the starting of the engine of the vehicle is available subsequent to the said perceptive test, whatever the level (N1, N2, N3) of risk presented by the user,
- and **in that** the method comprises for at least one level (N2, N3) of risk termed "risky" a step of starting the engine of the vehicle performed subsequent to the said perceptive test (120) on an intentional action of the user.

2. Method according to Claim 1, in which the said perceptive test is a sensory perception test.

3. Method according to Claim 1, in which the said perceptive test is a reaction time test.

4. Method according to any one of Claims 1 to 3, furthermore comprising a step consisting in associating with at least one level (N1, N2, N3) of risk at least one technical action for limiting use of the vehicle.

5. Method according to Claim 4, in which the said technical action relates to the immobilization of the vehicle.

6. Method according to Claim 5, in which the technical action relating to the immobilization of the vehicle is the disabling of the steering or the stalling of the engine.

7. Method according to any one of the preceding claims, in which for a level of risk (N1) termed "risk free" the starting of the engine of the vehicle is performed automatically (131) subsequent to the said perceptive test (120)

8. Method according to any one of Claims 1 to 7, in which the said intentional action consists in again making (132) a request to start the engine of the vehicle.

9. Method according to any one of Claims 1 to 8, furthermore comprising a test (160) of verification of identity between the driver of the vehicle and the user subjected to the said perceptive test (120).

10. Method according to Claim 9, in which the said verification test (160) relates, separately or in combination, to the occupancy of the driver's seat, the opening of the driver's door, the fastening of the driver's safety belt, the presence of the driver.

11. Method according to one of Claims 9 or 10, in which the said verification test (160) is performed automatically previously or in parallel with the said perceptive test (120), the vehicle having stopped.

12. Method according to Claim 11, in which in the case of negative verification test (160), the engine of the vehicle is stopped and/or the vehicle is immobilized (170).

13. Method according to any one of Claims 1 to 12, comprising an initial step consisting in providing a specific code for the compulsory execution of the perceptive test (120).

14. System for limiting use of a vehicle as a function of the state of vigilance of a user of the said vehicle, comprising:
- a test interface (11), able to subject the said user to at least one perceptive test,
- an information interface (14) able to inform the user of the level of risk that he presents,
- a central management unit (10), able to compare the result of the said perceptive test with a scale of levels (N1, N2, N3) of risk in relation to the driving of the vehicle and deduce therefrom the level of risk presented by the user, the said system **characterized in that** the said central management unit (10) is able to command the test interface (11) on receipt of a signal requesting starting of the engine of the vehicle, originating from a device (16) for starting the vehicle, and **in that** it comprises:
- a module (15) for verification of identity between the driver of the vehicle and the user subjected to the said perceptive test.

15. System according to Claim 14, in which the said central management unit (10) is also able to associate with at least one level of risk at least one technical action for limiting use of the vehicle.

16. System according to Claim 15, in which the said information interface (14) is also able to inform the user of the said technical action associated with the level of risk that he presents.

17. System according to one of Claims 15 or 16, furthermore comprising a command module (13) for controlling at least one member of the vehicle able to execute the said technical action associated with the level of risk presented by the user.

18. System according to any one of Claims 14 to 17, comprising a parametrization interface (12) able to receive a specific code for the compulsory execution of the perceptive test.
